# EUROPEAN PATENT SPECIFICATION

(11) **EP 1 720 509 B1**
(45) Date of publication and mention of the grant of the patent: **13.07.2011**
(21) Application number: 04783555.8
(22) Date of filing: 08.09.2004
(51) Int. Cl.: A61K 8/18

(54) **FOLDED SUBSTRATE WITH APPLIED CHEMICALS**
GEFALTETES SUBSTRAT MIT AUFGEBRACHTEN CHEMIKALIEN
SUBSTRAT PLIE SUR LEQUEL SONT APPLIQUES DES PRODUITS CHIMIQUES

(30) Priority: 17.12.2003 US 738269
(43) Date of publication of application: 15.11.2006
(73) Proprietor: KIMBERLY-CLARK WORLDWIDE, INC., Neenah, WI 54956 (US)
(72) Inventor: SOSALLA, Gerald, K., Appleton, WI 54911 (US); HOWELLS, Scott, D., Oshkosh, WI 54901 (US); SWIECICKI, Alethea, A.M., Greenville, WI 54942 (US); DECKER, Christopher, V., Appleton, WI 54913 (US)
(74) Representative: Mabey, Katherine Frances
(86) International application number: PCT/US2004/029343
(87) International publication number: WO 2005/067868

(56) References cited:
- EP-A- 0 983 739
- WO-A-03/008300
- WO-A-03/026473
- US-A- 5 540 332
- US-A- 5 607 754
- US-A- 6 030 331
- US-A- 6 121 165
- US-A- 6 136 775

## Description

### BACKGROUND

Often chemicals are applied to substrates to enhance their functionality. A wide variety of chemicals or lotions can be applied to a substrate, which is then folded and packaged for sale. For example, a surfactant can be applied to a nonwoven substrate and then the substrate dried of any moisture. The resulting wipe is suitable for cleaning surfaces, human skin, or hair, and eliminates the need to separately apply cleansers to the wipe prior to using, since the wipe is ready for use after wetting with water.

However, the chemical applied to the surface of the substrate may leave a residue on a person's skin or hands when handling the wipe. For example, removing a wipe from a larger package to take along for future use can leave an undesirable residue on hands. Furthermore, the chemical can leave a residue on other objects such as diaper bags or purses where an individual wipe may be placed after being removed from a larger package for future use. Therefore, a need exists for a substrate having an applied chemical that does not leave a residue during handling or contact with other objects and surfaces.

Additionally, the chemical applied to the substrate may interfere with proper dispensing of the substrate. For example, the chemicals applied to the wipe may have a slippery or oily feel that can make grabbing and removing an individual wipe difficult since the chemical can interfere with obtaining a good grip on the substrate. Other chemicals may have a tacky or sticky effect that can cause blocking where the individual wipes become stuck together interfering with proper dispensing. Therefore, a need exists for a substrate having an applied chemical that does not interfere with proper dispensing of the substrate.

Additionally, the chemical applied to the substrate may degrade the substrate in an unacceptable manner. For example, many substrates are laminates of two or more layers held together by an appropriate fastening means such as thermal bonding, adhesive/chemical, or other physical bonding, ultrasonic bonding or combinations thereof. The chemical applied to the wipe may degrade the adhesive weakening the bond strength or causing the substrate's layers to delaminate. Additionally, the applied chemical may change the surface texture of the substrate. For example, the chemical when coated onto the substrate may reduce or eliminate a desirable texture of the substrate. Therefore, a need exists for a substrate having an applied chemical that minimizes degradation of the substrate.

US 5,607,754 discloses a paper web product.

### SUMMARY

The inventors have discovered that If the chemical is applied to the substrate in discrete areas and then the substrate is folded in such a manner as to cause the discrete chemical areas to become interior surfaces after folding, chemical residues from the substrate can be minimized or eliminated. Furthermore, the inventors have discovered that by applying the chemical to discrete areas of the substrate, degradation of the substrate can be minimized or reduced. Additionally, the inventors have discovered that by including both a chemical free edge strip and a chemical free fold strip, further improvements in dispensing, preventing delamination, or residue reduction are possible. The present invention provides a product in accordance with claim 1.

### BRIEF DESCRIPTION OF THE DRAWINGS

The above aspects and other features, aspects, and advantages of the present invention will become better understood with regard to the following description, appended claims, and accompanying drawings in which:
Figure 1 illustrates one arrangement not in accordance with the invention.
Figure 2 illustrates the substrate of Figure 1 partially folded.
Figure 3 illustrates the arrangement of Figure 1 completely folded.
Figure 4 illustrates and embodiment of the invention.
Figure 5 illustrates the substrate of Figure 4 partially folded.
Figure 6 illustrates a package containing a plurality of folded substrates.
Figure 7 illustrates another arrangement not in accordance with the Invention partially folded.
Figure 8 illustrates another arrangement not in accordance with the invention partially folded.
Figure 9 illustrates another embodiment of the invention.

Repeated use of reference characters in the specification and drawings is intended to represent the same or analogous features or elements of the invention.

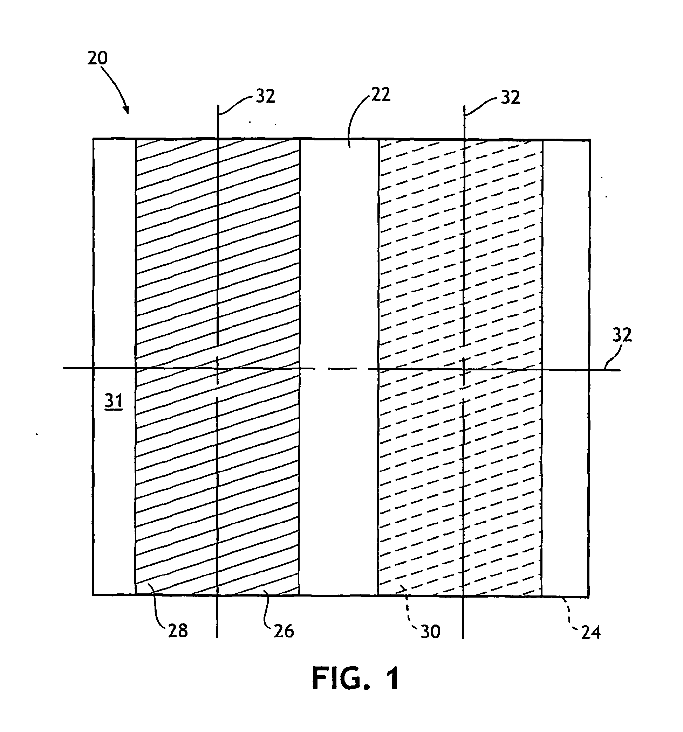

Figures 1,2,3,7 and 8 have been retained for the purposes of facilitating understanding of certain features of the Invention, but are not In accordance with the invention of claim 1.

As used herein forms of the words "comprise", "have", and "include" are legally equivalent and open-ended. Therefore, additional non-recited elements, functions, steps or limitations may be present in addition to the recited elements, functions, steps, or limitations.

As used herein "substrate" is a flexible sheet or web material, which is useful for household chores, personal care, health care, and cosmetic application or removal. Non-limiting examples of suitable substrates of the present invention include nonwoven substrates, woven substrates, hydro-entangled substrates, air-entangled substrates, paper substrates such as tissue, toilet paper, or paper towels, waxed paper substrates, coform substrates, wet wipes. Furthermore, laminated or plied together multi-layer substrates of two or more layers of any of the preceding substrates are suitable. In accordance with the present invention the folded substrate is a wipe.

Further examples of suitable substrates include a substantially dry substrate (less than 10% by weight of water) containing lathering surfactants and conditioning agents either impregnated into or applied to the substrate such that wetting of the substrate with water prior to use yields a cleansing product. Such substrates are disclosed in U.S. patent 5,980,931 entitled *Cleansing Products Having A Substantially Dry Substrate* issued to Fowler et al. on November 9, 1999.

Other suitable substrates may have encapsulated ingredients such that the capsules rupture during dispensing or use. Examples of encapsulated materials include those disclosed in U.S. patents 5,215,757 entitled *Encapsulated Materials* issued to El-Nokaly on June 1, 1993, and 5,599,555 entitled *Encapsulated Cometic Compositions* issued to El-Nokaly on February 4, 1997.

Other suitable substrates include dry substrates that deliver liquid when subjected to in-use shear and compressive forces. Such substrates are disclosed in U.S. 6,121,165 entitled *Wet-Like Cleaning Articles* issued to Mackey et al. on September 19, 2000.

As used herein "substantially dry" means that the substrate contains less than about 25 percent water as tested under ASTM D1744-92 entitled "Standard Test Method for Determination of Water in Liquid Petroleum Products by Karl Fischer Reagent" modified as follows: A 500 milligram ± 100 milligram sample is cut from the substrate and weighed on an analytical balance to the nearest 0.1 milligram. Adjust the size of the sample as needed to obtain the specified sample weight. Introduce the sample to the titration vessel and stir approximately 5 minutes to extract the water from the sample. After stirring the sample, titrate as described in the above test procedure and calculate the percent water as described in the above test procedure. In other embodiments of the invention, the substantially dry substrate can contain less than about 20 percent water, less than about 15 percent water, or less than about 10 percent water as tested above.

If the substrate is coated with a chemical or has variations In moisture content depending upon the sample location, a sufficient number of samples from all areas of the substrate should be tested and averaged together to establish within ± 1 percent the average moisture content for the entire substrate. For example, if the chemical coating comprises 30 percent of the surface area of the substrate, numerous samples should be taken from the substrate in both the coated and non-coated areas and tested. To establish the average moisture content of the entire substrate, 30 percent of the samples used In the final average should be from the coated area and the remaining 70 percent of the samples used in the final average should be from the uncoated area.

### DETAILED DESCRIPTION

It is to be understood by one of ordinary skill in the art that the present discussion is a description of exemplary embodiments only, and is not Intended as limiting the broader aspects of the present invention, which broader aspects are embodied in the exemplary construction.

Referring now to Figure 1, one arrangement of a substrate 20 having a first 22 and a second 24 opposing surface with a chemical 26 either applied onto, adjacent to or impregnated into the substrate is illustrated. The chemical is applied non-uniformly to the substrate such that it residues only in discrete areas with other discrete areas of the substrate free of the chemical. In the illustrated embodiment, the chemical is applied in a first chemical strip 28 located on the first surface 22 and in a second chemical strip 30 located on the second surface 24 as illustrated by the dashed lines. One means of achieving a non-uniform chemical application is to slot coat the chemical onto the first and second sides of the substrate.

The chemical is applies In such a manner as to leave a chemical free edge strip 31 present on the first and second surfaces. It is not necessary for the chemical free edge strip to run the entire length of the edge. For example, the chemical free edge strip could be intermittent along the edge with portions of the substrate adjacent the edge having the applied chemical and other portions free of the applied chemical.

One function or advantage of the chemical free edge strip is to provide a finger tab for grasping the substrate in order to open or remove the substrate from its packaging. By leaving at least a portion of the edge chemical free, it can be easier to grasp or open the substrate, especially when the applied chemical causes the substrate to become slippery, wet, or oily to the touch. Additionally, if the applied chemical is prone to blocking or sticking to the other surface when folded, the chemical free edge strip provides a free surface to grab that is not stuck, adhered, or held down. Another function or advantage of the chemical free edge strip is to prevent or minimize delamination, also known as ply separation, of a multi-layer substrate. As mentioned, the applied chemical can sometimes interfere with the bonding between layers of a multi-layer substrate. By leaving at least a portion of the edges of the substrate chemical free, the individual layers are less likely to delaminate. Ensuring a strong bond between the layers of the multi-layer substrate at the edges lessens or eliminates complete delamination of the layers even when other portions of the substrate become delaminated.

In various embodiments of the invention, the width of the chemical free edge strip 31 can be about 1 mm wide or greater, about 5 mm wide or greater, about 10 mm wide or greater, between about 1 mm to about 50 mm wide, between about 5 mm to about 25 mm wide, or between about 10 mm to about 20 mm wide

In various embodiments of the invention, the width of the chemical strip can be about 1 mm wide or greater, about 5 mm wide or greater, about 10 mm wide or greater, about 20 mm wide or greater, between about 1 mm to about 150 mm wide, between about 5 mm to about 100 mm wide, or between about 10 mm to about 50 mm wide.

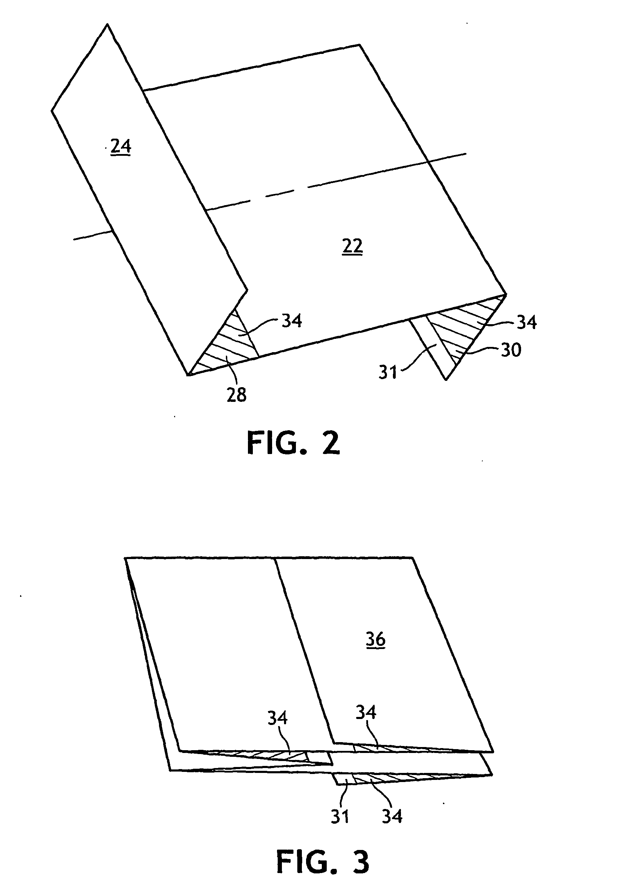

Illustrated in Figure 1 is a plurality of fold axes 32 that mark where the substrate is folded upon itself after applying the chemical strips. Referring now to Figures 2 and 3, the substrate of Figure 1 is illustrated in a sequence of folding steps. As seen in Figure 2, the substrate is first folded into a Z fold such that the first and second chemical strips become interior surfaces 34 after folding the substrate. As such, the chemical is no longer exposed on either the first or second surfaces of the substrate after folding. By folding the substrate such that the chemical is hidden from the exterior surfaces of the folded substrate, the possibility of the chemical leaving a residue on surfaces, or hands is minimized or eliminated when handling or storing the substrate. Thus, an individual wipe can be removed from a package and placed directly into a purse or diaper bag without worrying about the chemical leaving a residue or having to place the wipe into a plastic bag for storage.

Although not needed to hide the applied chemical, the substrate is then folded in half after Z folding as illustrated in Figure 3. The resulting folded substrate is approximately one-quarter the size of the original substrate before folding. The quarter fold, Z-folded substrate illustrated in Figure 3 is a convenient size for packaging and transport. Furthermore, the folded substrate is easily opened by simply grasping the chemical free edge strip 31 on an exterior folded panel 36 and shaking or allowing the weight of the substrate to open the other folds.

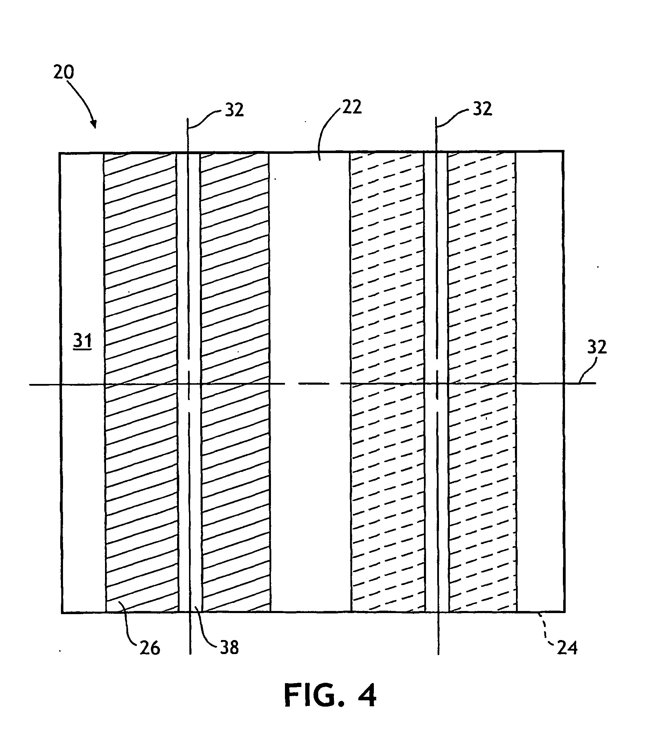

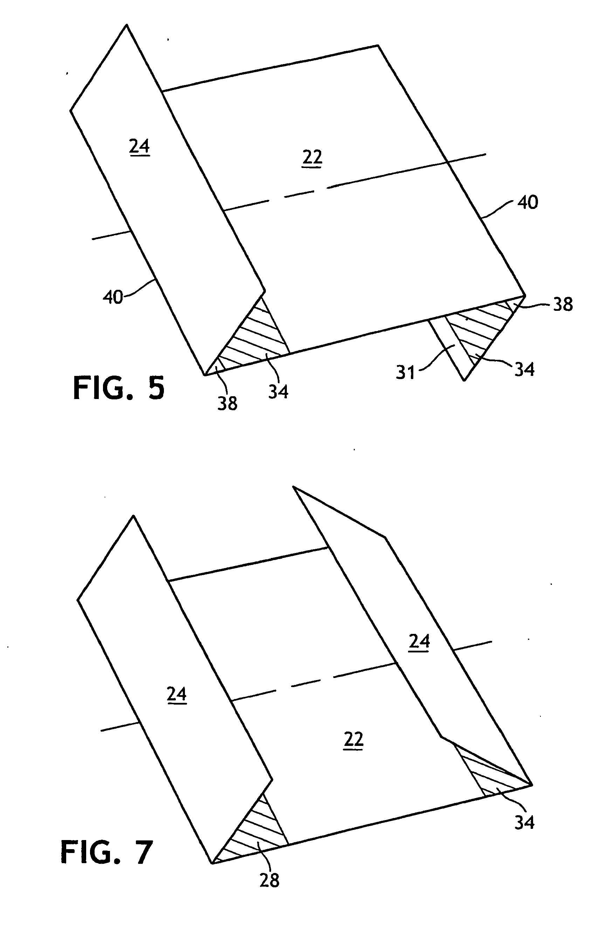

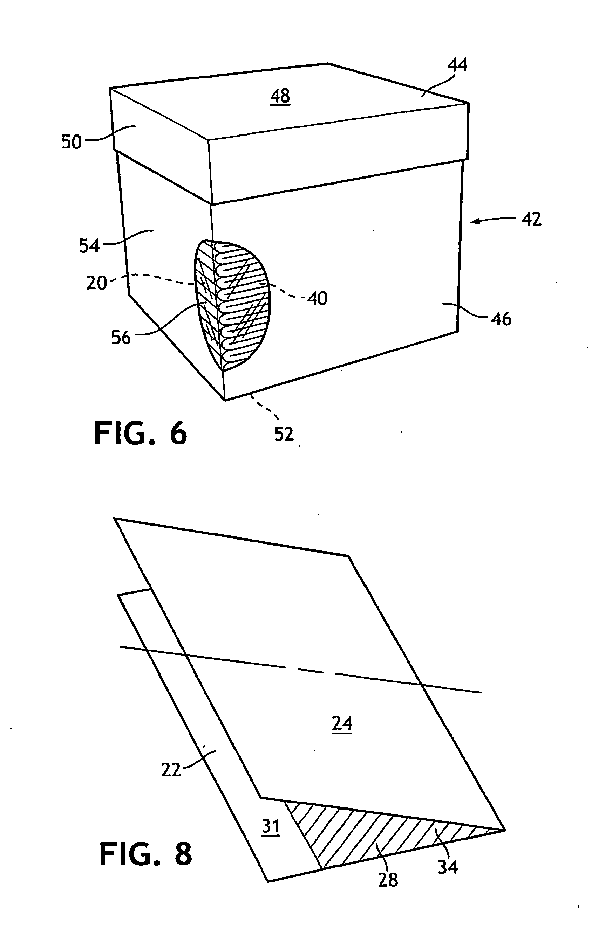

Referring now to Figures 4 and 5, an embodiment of the invention is illustrated. Instead of two chemical strips, the substrate 20 has four chemicals strips (two on the first side and two on the second Side) where the chemical is applied. Similar to the arrangement of Figure 1, the substrate has an edge strip 31 on each side that is left chemical free. Additionally, the substrate has at least one chemical free fold strip 38 that is left chemical free. The fold strip coincides with one or more of the substrate's fold axes 32. After folding as illustrated in Figure 6, the chemical free fold strip becomes a folded edge 40. As illustrated, the substrate can be folded along the chemical free fold strip such that the applied chemical becomes an interior surface after folding. The chemical free fold strip can help reduce chemical residue on surfaces that come into contact with the folded edge.

Referring now to Figure 6, a package 42 having a cover 44 and a body 46 is illustrated. In one embodiment, the package comprised a carton made from a paperboard or cardboard material. The package contains a plurality of folded substrates 20 having an applied chemical. The cover 44 includes a top 48 and a cover sidewall 50. The cover sidewall 50 in one embodiment includes four generally rectangular panels that intersect at approximately 90 degree angles. The body 46 includes a bottom 52, a body sidewall 54, and a clear window 56. The body sidewall 54 in one embodiment includes four generally rectangular panels that intersect at approximately 90 degree angles. The clear window 56 can be located anywhere in the package. In one embodiment, the clear window 56 spanned a corner of the body sidewall 54 such that a portion of the clear window was present on each of two intersecting panels of the body sidewall.

For arrangements not in accordance with the invention where the applied chemical after folding is not on the interior surfaces but instead resides on the exterior surfaces (not illustrated), it is still possible to have the edges of the substrate chemical free by use of the chemical free fold strip. Such an arrangement would help minimize any residue from the folded edges on the inside of a package containing a stack of several individual folded substrates. For example, if the package has a clear window for seeing into the interior of the package, the chemical free fold strip can prevent or minimize chemical residue being transferred onto the window that could reduce visibility through the window. Alternatively, the substrate can be folded with the applied chemical placed on an interior surface and placed into the package. If the applied chemical is prone to wicking through the substrate, the chemical free fold strip can prevent or minimize chemical residue on the window.

One of the functions or advantages of the chemical free fold strip is to further minimize or reduce the chances of the chemical leaving any residue on surfaces. For example, after folding, the individual substrates may be assembled into a stack, and the stack may be placed into a package such as a cardboard carton. During the folding process, the compressive forces exerted on the substrate can squeeze or transfer the chemical 26 from the interior surface 34 through the substrate to either the folded edge 40 or another portion of the exterior surface. When the folded substrate is placed into the carton, the folded edge can come into contact with the carton's interior surfaces. If the applied chemical is prone to wicking or migration, the chemical can leach from the folded edge of the substrate into the cardboard and become visible on the carton's interior surface, exterior surface, or clear window reducing the attractiveness of the packaging. A consumer may be hesitant to buy and/or use the packaged product with a stained interior, exterior, or window thinking the product is somehow damaged. By utilizing one or more chemical free fold strips 38 the problem is eliminated or reduced.

Another advantage of the chemical free fold strip is to minimize or reduce degradation of the substrate. For example, a plurality of chemical free fold strips can help prevent delamination of a multi-layer substrate by providing additional areas that are free of the applied chemical, which could interfere with bonding between the layers. Additionally, leaving areas of the substrate free of the applied chemical can allow for a different texture or physical surface feel between the chemical free areas and the areas where the chemical has been applied. Depending on the nature of the applied chemical, it can be desirable to leave uncoated areas of the substrate adjacent or near coated areas of the substrate.

In various embodiments of the invention, the width of the chemical free fold strip can be 1 mm wide or greater, 5 mm wide or greater, 10 mm wide or greater, between 1 mm to 50 mm wide, between 5 mm to 25 mm wide, or between 10 mm to 20 mm wide.

Referring now to Figure 7, an additional arrangement not in accordance with the invention is illustrated. Rather than a Z-folded substrate, the substrate can be C-folded as illustrated to position the applied chemical to the interior surfaces 34. Thereafter, the substrate may be optionally folded in half to become approximately one-quarter the size of the original substrate.

Referring now to Figure 8, an additional arrangement is illustrated. Rather than a Z-folded substrate, the substrate is V-folded, as illustrated, to position the applied chemical to the interior surfaces 34. Thereafter, the substrate may be optionally folded in half to become approximately one-quarter the size of the original substrate.

While the invention has been illustrated with C-, V-, and Z-folding and then optionally quarter-folding by folding the substrate in half, any manner of folding the substrate to hide the applied chemical from the exterior surfaces of the substrate after folding is possible. For example, after C-, V-, or Z-folding, the substrate could be folded three, four, or more times instead of just once in half as illustrated. Variations of C-, V-, or Z-folds are possible such that the folded portions of the substrate are not symmetrical after folding. For example, J- or S-folds are possible by changing the location of the fold axis.

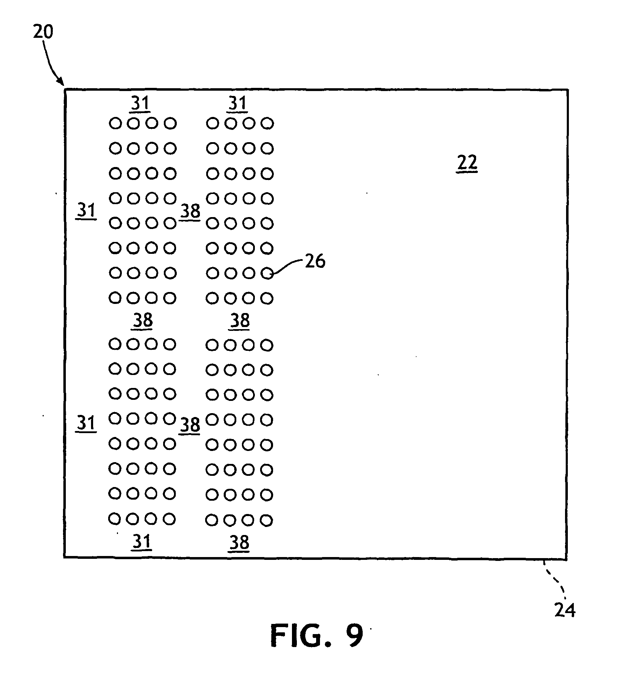

Referring now to Figure 9, another embodiment of the invention is illustrated. In this embodiment, the applied chemical residing on the second side 24 and the fold axes 32 are not illustrated for clarity. In the illustrated embodiment, the chemical 26 is sprayed, coated, or printed in a discrete pattern onto both sides of the substrate 20 by using a mask, a grating, a printing roll, or, other means to form four generally rectangular areas on each side of the substrate having the applied chemical. Instead of rectangles, other geometric shapes can be used or simply irregularly shaped patches.

The applied chemical does not extend to the edges of the substrate, such that the chemical free edge strip 31 runs around the entire perimeter of both sides of the substrate. The quadrant placement of the discrete chemical areas also ensures that for each of the three fold axes, there is a chemical free fold strip 38 present. Thus, after folding, all of the substrate's folded edges are unlikely to have any of the applied chemical present, reducing any chance of the chemical leaving a residue from contact with the folded edge. After applying the chemical to both the first and the second sides, the substrate can be folded as illustrated in Figures 2 and 3.

The chemical applied to the substrate can be any useful chemical or mixture of various chemicals that enhances the functionality of the substrate for its intended purpose. Possible chemical additives include, without limitation, strength additives, absorbency additives, softener additives, surfactant additives, conditioning additives, aesthetic additives such as fragrances or dyes. Other additives include, without limitation, anti-acne additives, antimicrobial additives, antifungal additives, antiseptic additives, antioxidants, cosmetic astringents, drug astringents, deodorants, detergents, emollients, external analgesics, binders, film formers, skin moisturizing ingredients as known in the art, opacifiers, skin conditioning agents, skin exfoliating agents, skin protectants, sunscreens, vapor rubs and the like. Suitable chemicals are disclosed in U.S. patent No. 5,400,403 issued to Troken et al. on Nov. 24, 1998, entitled *Multi-Elevational Tissue Paper Containing Selectively Disposed Papermaking Additive.* Additional suitable chemicals are disclosed in the previous references.

In one embodiment, the chemical applied to the substrate was a surfactant formulation comprising a concentrated (60 percent active ingredients) detergent system of a nonionic alkylpolyglucoside and zwitterionic amido betaine. High levels of a polyol, such as glycerin, allow the surfactant formulation to remain at a low viscosity for improved slot-coating capability during manufacturing. The lathering surfactants utilized in the surfactant formulation are Decyl Glucoside and Cocamidopropyl Betaine. Decyl Glucoside, from about 5 percent to about 40 percent of the total active ingredients of the surfactant formulation, is a mild, nonionic alkylpolyglucoside used for detergency and foam volume properties. Cocamidopropyl Betaine, from 0.5 percent to 25 percent of the total active ingredients of the surfactant formulation, is a high-foaming amphoteric detergent to deliver "quick" flash foam with minimal agitation upon dilution. Glycerin (a polyol), from about 0.5 percent to about 40 percent of the total active ingredients of the surfactant formulation, and PEG-7 Glyceryl Cocoate (a glyceryl ester), from 0.5 percent to 25 percent of the total active ingredients of the surfactant formulation, are both water-soluble conditioning agents or humectants/emollients designed to deliver moisture to the skin. Glycerin has a secondary function in the surfactant formulation as a diluent to lower the surfactant formulation's viscosity for improved slot-coating capability when applying the surfactant formulation to the substrate manufacturing. DMDM Hydantoin as a bactericide, about 0.4 percent of the total active ingredients of the surfactant formulation, and lodopropynyl Butylcarbamate as a fungicide, about 0.03 percent of the total active ingredients of the surfactant formulation, act together as a preservative system for the surfactant formulation. A fragrance (Shaw Mudge 62526M) containing lavender and chamomile extracts, from 0.1 percent to 1 percent of the total active ingredients of the surfactant formulation, provides a lavender and chamomile baby scent. The remaining component was approximately 40 percent water, which serves as a diluent or solvent to keep the surfactant formulation in a pourable/pumpable, fluid state. Other formulations can comprise from 30 percent to 90 percent active ingredients with the balance water.

The chemical may be applied onto, adjacent to, or impregnated into the substrate by any means known in the art. The chemical may also be placed between or adjacent to any of the layers within a multi-layer substrate, or applied to or impregnated into any of the layers. The chemical may be applied to the substrate, the substrate folded, and then the substrate allowed to dry after being packaged. Since the chemical can be placed onto an interior surface after folding, it is not necessary to dry the substrate prior to either folding or packaging, saving a processing step. Alternatively, the substrate can be dried after the chemical is applied, folded, and then packaged.

Suitable chemical application methods include, but are not limited to, flexographic printing, rotogravure printing, offset printing, letterpress, direct gravure coating, offset gravure coating, reverse roll coating, flexographic coating, slot coating, dip coating, rod coating, knife coating, air knife coating, blade coating, slide coating, curtain coating, spraying, hot melt spraying, foam application, and extrusion. Further information on coating methods is disclosed in Modem Coating and Drying, Edward Cohen and Edgar Gutoff, 1992 VCH Publishers, Inc.

The chemical may be added or applied to the substrate in any effective amount. The addition rate will depend to some degree on the chemical being applied and the type of substrate that the chemical is applied to. In various embodiments of the invention, the chemical addition rate can be between about 1 percent to about 400 percent based on the substrate's weight or between 10 percent to 200 percent based on the substrate's weight.

### EXAMPLE 1

A 115 gsm (grams per square meter) multi-layer substrate comprising three layers with an applied chemical was manufactured. Each of the substrate's two outer surface layers comprised a 34.5 gsm coform material having 60 percent pulp fiber identified as CF405 fiberized southern softwood pulp available from Weyerhauser and 40 percent polymeric fibers identified as PF-015 polypropylene meltblown available from Basell. The substrate's inner layer comprised a 23 gsm elastomeric material comprised of a mixture of polyethylene materials that are sold by Dow Chemical. Seventy percent (70%) of the inner layer comprised Dow Affinity XUS59400.03L, a metallocene-catalyzed polyethylene. Thirty percent (30%) of the inner layer comprised a mixture of 80 percent Dow Affinity XUS59400.03L, 15 percent Regalrez 1128 Tackifier, and 5 percent Dow DNDN 1077, a linear low density polyethylene.

The substrate's outer surface layers and inner layer were laminated together. The inner layer is stretched by approximately 2.2 times its original length and then laminated to the outer layers using an embossing pattern. The multi-layer substrate is then allowed to retract approximately 30 percent to achieve a final basis weight of approximately 115 gsm. U.S. patent application 09/751,329 entitled *Composite Material With Cloth-Like Feel* filed on December 29, 2000, provides more details on the process used to make the multi-layer substrate. Another multi-layer substrate is disclosed in U.S. patent no. 4,720,415 entitled *Composite Elastomeric Material and Processing for Making the Same* issued to Vander Wielen et al. on January 19, 1988.

After the substrate was made, the substrate was slot coated with four chemical strips (two on each side of the substrate) as shown in Figure 4. The substrate measured approximately 216 mm in width by 216 mm in length. The chemical free edge strips on the first and second opposing surfaces were approximately 12 mm wide. The four chemical strip widths measured approximately 36 mm wide. The chemical free fold strips between the chemical strips measured approximately 12 mm wide. The chemical free strip between the inner two chemical strips on opposite sides of the substrate was approximately 24 mm wide.

The above recited surfactant formulation was applied at a rate of about 4 grams per sheet. After slot coating, the substrate was folded as illustrated in Figure 5 and then quarter-folded as illustrated in Figure 3. The folded substrate was stacked with other identically prepared folded substrates. A stack of approximately fourteen (14) individually folded substrates was then packaged into a paperboard carton as illustrated in Figure 6. The folded substrates were allowed to dry due to evaporation occurring during manufacturing and from the carton after packaging. Due to either evaporation or by applying a reduced moisture content formulation, the coated substrate can be a substantially dry substrate for certain applications. If desired, the interior of the carton can be coated with a coating to make the carton more impervious to liquids during the drying phase or to provide increased resistance to the chemicals contained in the formulation.

The substrate produced by the above process is useful for a disposable washcloth. By placing the washcloth in water, the surfactant formulation is activated. The chemical free edge strip enabled ready dispensing by providing a chemical free edge to grab when removing the wipe. The folding method ensured that the applied chemical became interior surfaces, thereby reducing any chemical residue on hands or surfaces in contact with the wipe prior to activating the applied chemical. The chemical free fold strip further reduced any chemical residue on surfaces such as the interior surfaces, the exterior surfaces, or the clear window of the paperboard carton used as a package. By folding the substrate with the applied chemical towards the interior surfaces, the chances of the individual folded substrates sticking together within the packaging were also minimized.

After slot coating with the applied surfactant formulation, the multi-layer substrate was tested for its delamination force as tested by the Small Angle Peel Test. In areas of the substrate having the applied chemical, the delamination force as tested below was determined to be approximately 2.5 g/cm. In areas of the substrate free of the applied surfactant formulation, the delamination force was determined to be approximately 45.4 g/cm. Even though the delamination force was found to be relatively low due to the applied surfactant formulation, the multi-layer substrate did not delaminate in use. The inventive chemical application pattern and utilization of the chemical free edge strips and fold strips helped to ensure that the multi-layer substrate remained intact during use.

### TEST METHODS

### Small Angle Peel Test

All testing is done in a standard laboratory atmosphere of 23 +/- 1 °C and 50 +/- 2% relative humidity. All test specimens must be conditioned for at least 4 hours prior to testing.

The Small Angle Peel Test measures the amount of force required to delaminate one layer from another layer in a multi-layer substrate. The test is conducted by delaminating a portion of the substrate by hand, inserting a peel arm test fixture between the delaminated layers, and then measuring the peak force needed to further delaminate the substrate using a tensile tester. To determine the small angle peel force, a tensile tester is utilized such as a Sintech tensile tester manufactured by Sintech Inc., Research Triangle Park, N.C. 27709.

The peel arm test fixture is a C-shaped test fixture having an upper delaminating arm and a lower base leg that is constructed from 0.25" (6.35 mm) thick stainless steel. The base leg of the test fixture is 0.75" high (19.0 mm) by 3.5" long (88.9 mm). A small plate can be T welded to the top of the base leg to ensure that the test fixture is aligned parallel to the lower jaws of the tensile tester by resting the plate against the top of the jaws and then clamping the jaws together. The upper delaminating arm is 0.38" high (9.65 mm) by 4.25" long (108 mm). The upper delaminating arm is beveled for a length of 3.25" (82.6 mm) from its free end to a symmetric V shape with the point of the V facing the base leg. The overall height of the test fixture is 4.84" (122.9 mm) and the width of the vertical support bracket supporting the upper delaminating arm above and parallel to the base leg is 0.75" (19.05 mm).

Insert the peel arm test fixture into the lower jaws of the tensile tester ensuring that the upper delaminating arm is parallel to the upper jaw. The gage length between the upper arm and the upper jaws of the tensile tester is set to 1.0 inches (25.4 mm).

The test specimen is cut to 1.0 cm (0.4") in width along the machine direction. After cutting the test specimen, delaminate the test specimen by hand for about 1.5" (38.1 mm). If necessary to ensure that a test strip having the applied chemical and a chemical free test strip can be tested, a narrower test strip can be utilized. Ensure that the layer having the applied chemical is delaminated from the rest of the multi-layer substrate. In the event that multiple chemicals are applied to multiple layers, the force required to separate each layer from an adjacent layer is separately tested.

Place the test specimen into the tensile tester by clamping the delaminated portion in the upper jaws after inserting the upper delaminating arm of the test fixture between the separated layers. Calibrate the tensile tester and load cell according to the manufacturer's directions. The maximum capacity of the load cell should be chosen such that the majority of the measured peak tensile values fall between 10% and 90% of the load cell's capacity. Ensure that the crosshead speed of the tensile tester is set to 0.25 inch per minute (6.35 mm/min), the break sensitivity is set to 95%, and the extension limit high is set to 3.5 inches (88.9 mm).

During the test, the upper jaws of the tensile tester pull the test specimen upwards causing further delamination of the test specimen as it is split in two by the upper delaminating arm of the test fixture. The upper jaw moves a total of 3.5 inches (88.9 mm) while the force data from the load cell is measured. The maximum load for each specimen at the test speed during the 3.5 inches of delamination is determined using a data acquisition program having a sufficient sampling rate to accurately record the maximum load. At least five (5) or more samples are tested to obtain a reliable average for the maximum load. The delamination force is calculated by dividing the average maximum load in grams by the test specimen width in centimeters to obtain the average delamination force expressed in g/cm.

## Claims

1. A product comprising:
a substrate (20) having a first (22) and a second (24) opposing side;
a discrete chemical area applied onto, adjacent to, or impregnated into *the* first and the second opposing side;
**characterized in that** the substrate comprises:
a chemical free fold strip (38); and
wherein the substrate (20) is folded along the chemical free fold strip (38) forming a folded edge, the substrate comprising: ,
two chemical strips (28) on the first side (22) separated by a chemical free fold strip; and
two chemical strips (30) on the second side (24) separated by a chemical free fold strip;
two chemical free edge strips (31), with one of the chemical free edge strips (31) adjacent each of the outer most chemical strips on the first and second sides;
and
wherein the substrate is Z folded along the chemical free fold strips such that all four chemical strips become interior surfaces,
or wherein the substrate is Z folded along the chemical free fold strips and
then the substrate is quarter folded such that all four chemical strips (28, 30) become interior surfaces;
and wherein the folded substrate is a wipe.

2. The product of claim 1
wherein a discrete chemical area is not applied to areas of the substrate that become
exterior surfaces after folding.

3. The product of any preceding claim wherein the substrate comprises a multi-layer substrate and the delamination force for the chemical free edge strip (31) is greater than or equal to the delamination force for the discrete chemical area.

4. The product of any preceding claim wherein the substrate comprises a multi-layer substrate and the delamination force for the chemical free fold strip (38) is greater than or equal to the delamination force for the discrete chemical area.

5. The product of any preceding claim wherein the width of the chemical free edge strip (38) is between 1 mm to 50 mm wide.

6. The product of claim 5 wherein the width of the chemical free fold strip (38) is between 1 mm to 50 mm.

7. The product of any preceding claim wherein the substrate is substantially dry.

8. The product of any preceding claim comprising a plurality of the folded substrates contained in a package (42) comprising a clear window (56)

9. The product of claim 8 wherein the package (42) comprises a carton having a cover (44) and a body (46) and the clear window (56) is located in the body.

10. The product of claim 9 wherein the body (46) comprises a body sidewall (54) and a bottom (52), the body sidewall comprised of four generally rectangular panels intersecting at approximately 90 degree angles, and wherein the clear window (56) spans a corner of the body sidewall such that a portion of the clear window is present on each of two intersecting panels of the body sidewall.

## Patentansprüche

1. Produkt umfassend:
ein Substrat (20), das eine erste (22) und eine zweite (24) entgegengesetzte Seite aufweist;
einen eigenständigen chemiekalienbereich, der auf die erste und zweite entgegengesetzte Seite aufgebracht ist, daneben liegt oder darin hinein imprägniert ist;
**dadurch gekennzeichnet, dass** das Substrat Folgendes umfasst:
einen chemikalienfreien Falzstreifen (38); und
wobei das Substrat (20) dem chemikalienfreien Falzstreifen (38) entlang unter Bildung einer gefalzten Kante gefalzt wird, wobei das Substrat Folgendes umfasst:
zwei Chemikalienstreifen (28) auf der ersten Seite (22), die durch einen chemikalienfreien Falzstreifen getrennt sind; und
zwei Chemikalienstreifen (30) auf der zweiten Seite (24), die durch einen chemikalienfreien Falzstreifen getrennt sind;
zwei chemikalienfreie Kantenstreifen (31), wobei einer der chemikalienfreien Kantenstreifen (31) neben jedem der äußersten Chemikalienstreifen auf den ersten und zweiten Seiten liegt; und
wobei das Substrat den chemikalienfreien Falzstreifen entlang Z-gefalzt ist, derart, dass alle vier Chemikalienstreifen Innenflächen werden,
oder wobei das Substrat den chemikalienfreien Falzstreifen entlang Z-gefalzt und das Substrat daraufhin viertelgefalzt wird, derart, dass alle vier Chemikalienstreifen (28, 30) Innenflächen werden;
und wobei das gefalzte Substrat ein Wischtuch ist.

2. Produkt nach Anspruch 1, wobei der eigenständige chemiekalienbereich nicht auf Bereiche des Substrats aufgebracht ist, die nach dem Falzen zu Außenflächen werden.

3. Produkt nach einem der vorhergehenden Ansprüche, wobei das Substrat ein Mehrschichtsubstrat umfasst und die Delaminationskraft für den chemikalienfreien Kantenstreifen (31) höher als oder gleich der Delaminationskraft für den eigenständigen chemischen Bereich ist.

4. Produkt nach einem der vorhergehenden Ansprüche, wobei das Substrat ein Mehrschichtsubstrat umfasst und die Delaminationskraft für den chemikalienfreien Falzstreifen (38) höher als oder gleich der Delaminationskraft für den eigenständigen chemischen Bereich ist.

5. Produkt nach einem der vorhergehenden Ansprüche, wobei die Breite des chemikalienfreien Kantenstreifens (38) zwischen 1 mm und 50 mm liegt.

6. Produkt nach Anspruch 5, wobei die Breite des chemikalienfreien Falzstreifens (38) zwischen 1 mm und 50 mm liegt.

7. Produkt nach einem der vorhergehenden Ansprüche, wobei das Substrat im Wesentlichen trocken ist.

8. Produkt nach einem der vorhergehenden Ansprüche, umfassend mehrere der gefalzten Substrate, die in einer Packung (42) enthalten sind, die ein klares Fenster (56) umfasst.

9. Produkt nach Anspruch 8, wobei die Packung (42) einen Karton umfasst, der einen Deckel (44) und einen Körper (46) aufweist und das klare Fenster (56) sich im Körper befindet.

10. Produkt nach Anspruch 9, wobei der Körper (46) eine Körperseitenwand (54) und einen Boden (52) umfasst, wobei die Körperseitenwand vier allgemein rechteckige Platten umfasst, die sich in Winkeln von etwa 90 Grad überschneiden, und wobei das klare Fenster (56) eine Ecke der Körperseitenwand überspannt, derart, dass ein Abschnitt des klaren Fensters auf jeder von zwei sich überschneidenden Platten der Körperseitenwand vorliegt.

## Revendications

1. Produit comprenant :
un substrat (20) ayant une première (22) et une seconde (24) faces opposées ;
une zone individualisée avec agent chimique, appliquée sur, adjacente à, ou imprégnant les première et seconde faces opposées ;
**caractérisé en ce que** le substrat comprend :
une bande de pli sans agent chimique (38) ;
le substrat (20) étant plié le long de la bande de pli sans agent chimique (38) en formant un bord plié, et le substrat comprenant :
deux bandes avec agent chimique (28) sur la première face (22), séparées par une bande de pli sans agent chimique ; et
deux bandes avec agent chimique (30) sur la seconde face (24) séparées par une bande de pli sans agent chimique ;
deux bandes de bord sans agent chimique (31), avec une des bandes de bord sans agent chimique (31) adjacente à chacune des bandes avec agent chimique les plus extérieures sur les première et seconde faces ;
produit dans lequel
le substrat est plié en Z le long des bandes de pli sans agent chimique de telle sorte que les quatre bandes avec agent chimique deviennent des surfaces intérieures, ou
le substrat est plié en Z le long des bandes de pli sans agent chimique, après quoi le substrat est plié en quatre de telle sorte que les quatre bandes avec agent chimique (28,30) deviennent des surfaces intérieures,
le substrat plié étant une lingette.

2. Produit selon la revendication 1, dans lequel une zone individualisée avec agent chimique n'est pas appliquée à des zones du substrat qui deviennent des surfaces extérieures après pliage.

3. Produit selon l'une quelconque des revendications précédentes, dans lequel le substrat comprend un substrat à couches multiples et dans lequel la force de déstratification pour la bande de bord sans agent chimique (31) est supérieure ou égale à la force de déstratification pour la zone individualisée avec agent chimique.

4. Produit selon l'une quelconque des revendications précédentes, dans lequel le substrat comprend un substrat à couches multiples et dans lequel la force de déstratification pour la bande de pli sans agent chimique (38) est supérieure ou égale à la force de déstratification pour la zone individualisée avec agent chimique.

5. Produit selon l'une quelconque des revendications précédentes, dans lequel la largeur de la bande de bord sans agent chimique (38) est comprise entre 1 mm et 50 mm.

6. Produit selon la revendication 5, dans lequel la largeur de la bande de bord sans agent chimique (38) est comprise entre 1 mm et 50 mm.

7. Produit selon l'une quelconque des revendications précédentes, dans lequel le substrat est sensiblement sec.

8. Produit selon l'une quelconque des revendications précédentes, comprenant une pluralité de substrats pliés contenus dans un conditionnement (42) comportant une fenêtre transparente (56).

9. Produit selon la revendication 8, dans lequel le conditionnement (42) comprend un carton ayant un couvercle (44) et un corps (46), et la fenêtre transparente (56) est située dans le corps.

10. Produit selon la revendication 9, dans lequel le corps (46) comprend une paroi latérale de corps (58) et un fond (62), la paroi latérale de corps étant composée de quatre panneaux généralement rectangulaires, sécants selon des angles d'approximativement 90°, et dans lequel la fenêtre transparente (56) s'étend sur un coin de la paroi latérale de corps de telle sorte qu'une portion de la fenêtre transparente est présente sur chacun de deux panneaux sécants de la paroi latérale de corps.
